# EUROPEAN PATENT SPECIFICATION

(11) **EP 3 500 756 B1**
(45) Date of publication and mention of the grant of the patent: **20.05.2020**
(21) Application number: 17745166.3
(22) Date of filing: 24.07.2017
(51) Int. Cl.: F04B 43/04, F04B 43/02, A24F 47/00, A61M 11/00, A61M 15/00, A61M 15/06

(54) **AEROSOL-GENERATING DEVICE**
AEROSOLERZEUGUNGSVORRICHTUNG
DISPOSITIF DE GÉNÉRATION D'AÉROSOL

(30) Priority: 16.08.2016 EP 16184283
(43) Date of publication of application: 26.06.2019
(60) Divisional application: 20169406.4
(73) Proprietor: Philip Morris Products S.A., 2000 Neuchâtel (CH)
(72) Inventor: MAZUR, Ben, Ashley Down Bristol BS7 9QP (GB)
(74) Representative: Ponder, William Anthony John
(86) International application number: PCT/EP2017/068691
(87) International publication number: WO 2018/033347

(56) References cited:
- US-A1- 2002 114 716
- US-A1- 2004 000 843
- US-A1- 2009 242 060
- US-A1- 2011 005 606
- US-A1- 2015 117 842
- US-A1- 2016 213 068

## Description

The invention relates to aerosol-generating devices, in particular to aerosol-generating devices comprising a micropump.

In aerosol-generating devices a liquid is vaporized or atomized in an atomizer, for example a heating element, arranged next to an opening of a cartridge comprising the liquid. In general, the liquid is transported to the atomizer by capillary action of a capillary material. However, in such systems the transport of the liquid is limited to short distances thus limiting an arrangement of cartridge and atomizer.

US 2015/0117842 A1 discloses an aerosol delivery device comprising a cartridge including a reservoir at least partially filled with an aerosol precursor composition. The aerosol delivery device further comprises an atomizer and a pump. The pump is arranged to dispense the aerosol precursor composition from the reservoir.

There is need for aerosol-generating devices providing more flexibility in the arrangement of a reservoir for aerosol-forming substrate and an atomizer for atomizing the aerosol-forming substrate. There is also need for such devices providing good performance in particular for viscous aerosol-forming fluids.

According to the invention there is provided an aerosol-generating device. The device comprises a cartridge for holding an aerosol-forming substrate and an atomizer for atomizing aerosol-forming substrate. The device further comprises a micropump for delivering a fluid, wherein the micropump is arranged between the cartridge and the atomizer and in fluid connection with the cartridge and the atomizer for supplying aerosol-forming substrate from the cartridge to the atomizer. The micropump comprises two pump chambers having two separate chamber volumes and two actuators, each actuator assigned to one of the two pump chambers for changing a respective chamber volume. Each pump chamber is provided with at least one inlet valve and at least one outlet valve for establishing a pumping direction. The micropump further comprises a common inlet and a common outlet. The two pump chambers are arranged in parallel and are in fluid connection with the common inlet and the common outlet, and the actuators are configured to operate in parallel such that a volume change in each of the two pump chambers occurs simultaneously for both pump chambers. For example a control electronics present in the aerosol-generating device may control the micropump or the actuators of the micropump respectively.

By operating the actuators simultaneously, and preferably having same volume changes by an actuator stroke, a pumping pressure may basically be doubled compared to having only one pumping chamber. A pumping pressure may also be doubled compared to for example, two pumps connected in series. Such single or serial micropumps are known, for example, from Bartels Mikrotechnik GmbH, with the Bartels mp5 micropump having one actuator and the Bartels mp6 micropump having two actuators in two serially arranged pump chambers.

Arranging two pump chambers in parallel also allows to provide high performance of the micropump and high flow rates also for viscous fluids. Since maximum flow rates typically decrease with higher viscosity fluids, two pump chambers at higher pumping pressure allow for well-defined high flow rates also for viscous or high viscous fluids.

A parallel arrangement of two pump chambers also facilitates a compact and small design of the micropump and of an aerosol-generating device the micropump is used in. This is in particular suitable for hand-held devices, where space is limited and the device should be miniaturized. Such hand-held devices may, for example, be inhalators for medical purposes or vaping or smoking devices for smoking purposes. Such hand-held devices may, for example, be vaping devices wherein an e-liquid is vaporized, such e-liquids typically being viscous fluids.

The actuators of the micropump may be driven by a control electronics connected to the micropump. The control electronics may be combined with a control electronics of the aerosol-generating device for controlling the device.

Actuators may be piezo membrane actuators, mechanical actuators, thermal actuators, magnetic actuators or other suitable actuators resulting in a volume change of a pump chamber. Preferably, disk- or plate-shaped actuators are used. In the device according to the invention, preferably, two piezo membrane actuators are used in the micropump.

In piezo membrane actuators, the amplitude of the voltage applied to the actuator defines the strokes of the actuator and therefore the displacement of the pumped medium per pump cycle. With rising amplitude of a controller voltage, the flow rate rises linearly to a maximum flow rate.

The flow rate also increases linearly in a defined frequency range. The frequency determines the number of pump strokes per unit of time. After reaching a maximum flow rate at the resonance frequency, the flow rate decreases again with higher frequencies above the resonant point.

The combination of signal, amplitude and frequency defines the performance of the micropump. Operation parameters may be adapted accordingly to the fluid to be transported by the micropump.

Each pump chamber of the micropump comprises at least one inlet valve and at least one outlet valve. Preferably, each pump chamber comprises two inlet valves and two outlet valves. Two valves per pump chamber have shown to provide a high reliability of operation of the micropump.

In operation, the fluid flows along a fluid path from the common inlet towards the common outlet, through each of the two pump chambers via their respective inlet valves and outlet valves. That is, the fluid path can be defined as a route or a trajectory a fluid follows as it is being transported in the micropump. Preferably the valves are arranged and designed in such a manner that direction changes of a fluid stream along the fluid path being invoked by the valves are minimized. Thus, energy for the transport of the fluid is minimized and additionally, the settling of gas bubbles is reduced. Moreover, a smoother fluid path reduces turbulence and lessens the amount of fluid impingement upon the walls of the micropump, and thus helping to reduce the amount of mechanical vibration and noise experienced by the user. Preferably, changes of angles in the fluid path are not abrupt. Preferably, the fluid path comprises no angles of 90 degree or smaller. For example, the design of the pump chambers and their respective valves ensures the fluid flows through the valves at an obtuse angle. Advantageously the direction of flow of fluid from the common inlet to the common outlet, along the fluid path, does not change by 90 degrees or more. In addition, holes in device elements of the micropump connecting different fluid planes comprise diameters preferably equal to or larger than a length of the holes.

Advantageously, the two pump chambers are in direct fluid connection with the common inlet and with the common outlet. Thus, each pump chamber is directly connected to the common inlet and the common outlet without intermediate fluid channels. Thus, advantageously, the fluid connections of the pump chambers and common inlet or common outlet are separated solely by the at least one inlet valve or the at least one outlet valve, respectively, of the pump chambers.

In the device the two pump chambers as well as the two actuators may be arranged opposite each other. Preferably, the two actuators and the two pump chambers are arranged exactly opposite each other. Such an arrangement facilitates the manufacture of the micropump and allows for the manufacture of a very compact micropump.

The two pump chambers and the two actuators may be configured such that a same volume change in each of the two pump chambers occurs upon operation of the two actuators.

The chamber volumes of the two pump chambers do not have to have a same size and may be different. However, preferably, the chamber volumes of the two pump chambers are identical.

In preferred embodiments, the components which are required for the production of one pump chamber of the micropump, particularly the valve(s) and the actuator are designed substantially interchangeable with, or identical, as the components for the second pump chamber of the same micropump. That is, preferably all actuators, valves and optionally other components that are present in each pump chamber are designed identical. By this, the risk of confusion during assembly is reduced and the production cost may be minimized.

Preferably, the micropump comprises a symmetric set-up in view of a plane arranged parallel to and between the two pump chambers.

A symmetric and in particular an identical set-up of the two pump chambers allow for a simplified control and operation of the micropump.

This symmetric set-up may include the common inlet and common outlet. Thus an inlet connection of the common inlet may be arranged at one side of the micropump and an outlet connection of the common outlet may be arranged at an opposite side of the micropump. However, an inlet connection of the common inlet and an outlet connection of the common outlet may also be arranged at a same side of the micropump. This allows the manufacture of an even more compact micropump.

Preferably, all components which are in contact with the fluid consist of the same material. Preferably the pump chambers, valves and common inlet and outlet are manufactured from a same material. Such a material may be adapted to the physical and chemical characteristics of a fluid to be pumped. For example, all components which are in contact with the fluid may consist of polyphenylene sulphone (PPSU). This material provides advantages with regard to the joining and fabrication technique preferably used for the joining of the micropump in the device according to the present invention. However, also other suitable materials may be used, for example polypropylene (PP) or polyimide (PI). It may be necessary, for example in the medical field to coat the parts that are in contact with the fluid, for example, by biocompatible or other particularly inert materials, in order to adapt the device to the specific requirements. Also other materials such as silicon, metals, or glasses may be used, wherein it must be ensured in the case of materials having only a low stretchability that the actuators can still lead to a change of the chamber volume.

All components of the micropump that must be joined to each other can for example be joined by suitable adhesives or preferably using laser welding. The latter technique is suitable in particular for the joining of plastics and offers, besides short production times, the possibility to produce a hermetically tight connection without adhesives that comes close to the strength of the source material.

In a preferred embodiment the micropump comprises the following elements, which sequence basically corresponds to an assembly sequence:
- two base elements each comprising a recess and half of an inlet and half of an outlet;
- two actuators with electrodes and electric terminals, wherein each of the actuators is arranged in one of the recesses;
- two protection layers arranged over each of the actuators each forming one side of a pump chamber;
- valve foils which are insertable into the recesses and which carry the movable parts of the inlet and outlet valves of the pump chambers;
- two intermediate layers which are also insertable into the recesses and which have openings which form the immobile parts of the inlet and outlet valves. The intermediate layers form together with the respective protection layers and recess side walls the pump chambers.

Actuator and protection foil as well as valve foil and intermediate foil may be kept in place by seals, for example by weld seals or an adhesive seal.

Upon positioning of a flow separation element at a position in between the inlet and outlet and in between the two base elements, the two base elements may be put together and assembled. Preferably, the two base elements are provided with a fluid-tight sealing, preferably by laser welding, and close the recesses of the two base elements in such a manner that the components in the interior of the base elements are protected from environmental influences. Upon assembly, each of the two halves of the inlet and outlet are also combined and form the common inlet and common outlet and corresponding inlet connection and outlet connection. These allow to connect the micropump to a reservoir and to the atomizer or to corresponding tubings connected to the reservoir and to the atomizer, respectively.

The aerosol-generating device may further comprise a flow sensor for controlling a fluid flow in the common outlet of the micropump. The flow sensor is connected to a control electronics. By this, a fluid flow may be controlled, for example, may be kept constant, for example by changing the micropump parameters if required. The device may thus comprise a controlled loop system.

The atomizer of the device may be designed to atomize or vaporize the aerosol-forming substrate by any suitable means. For example, the atomizer may vaporize a substrate by heat, atomize or nebulize a substrate by ultrasound or other vibration means. The atomizer in the device according to the invention may comprise any one or a combination of an acoustic atomization element, an ultrasonic vibrator, a vaporizer such as a heater or any other atomizer suitable for atomizing aerosol-forming substrate.

The device may comprise any fluid to be atomized. The fluid may be gas or liquid or a combination thereof. Preferably, the aerosol-generating substrate is a liquid. However, the aerosol-generating substrate may originally also be a solid, which is liquefied, for example by heating, such that the liquid may be transported by the micropump to the atomizer.

The aerosol-forming substrate may, for example, comprise medicine, flavour or stimulating substances.

Liquid aerosol-forming substrate may comprise at least one aerosol former and a liquid additive.

The aerosol-former may, for example, be propylene glycol or glycerol.

The liquid aerosol-forming substrate may comprise water.

Preferably, the aerosol-forming substrate is an e-liquid to be used in vaping systems.

In such systems, the liquid additive may be any one or a combination of a liquid flavour or liquid stimulating substance. Liquid flavour may, for example, comprise tobacco flavour, tobacco extract, fruit flavour or coffee flavour. The liquid additive may, for example, be a sweet liquid such as for example vanilla, caramel and cocoa, a herbal liquid, a spicy liquid, or a stimulating liquid containing, for example, caffeine, taurine, nicotine or other stimulating agents known for use in the food industry.

The device preferably comprises one or a combination of nicotine containing aerosol-forming substrate and tobacco flavour containing aerosol-forming substrate.

Preferably, the device according to the invention comprises a viscous liquid aerosol-forming substrate comprising a viscosity between 1 mPas and 200 mPas, preferably between 1 mPas and 150 mPas, for example between 80 mPas and 130 mPas.

Preferably, the aerosol-generating device is an electronic smoking device as used in electronic smoking systems. The smoking device may be a hand-held device. The smoking system may be a smoking system wherein tobacco is heated rather than combusted.

Exemplary values for the micropump preferably used in the device according to the invention are:
- A chamber volume between 1 ml and 2 ml;
- A flow rate between 1 µL/s and 7 µL/s;
- A pumping pressure between 500 mBar and 700 mBar;
- A size of the micropump (without connectors) about 14x14x6. 5mm³;

Exemplary operation parameters of the micropump are:
- Frequencies between 0 and 300 Hz.
- Voltages up to maximal 320Vpp.
- A sinusoidal, rectangular or intermediately shaped actuator voltage curve. Preferably, lifting and lowering phases of the actuator are not identical. Preferably, an actuator voltage curve is adapted in order to optimize the micropump in view of noise and bubble generation at a given flow rate and viscosity of a fluid.

The invention is further described with regard to embodiments, which are illustrated by means of the following drawings, wherein:
- Fig. 1: is an exploded view of a set-up of a micropump having two serially arranged actuators showing exemplary parts of the micropump;
- Fig. 2: shows a micropump with parallel actuators.

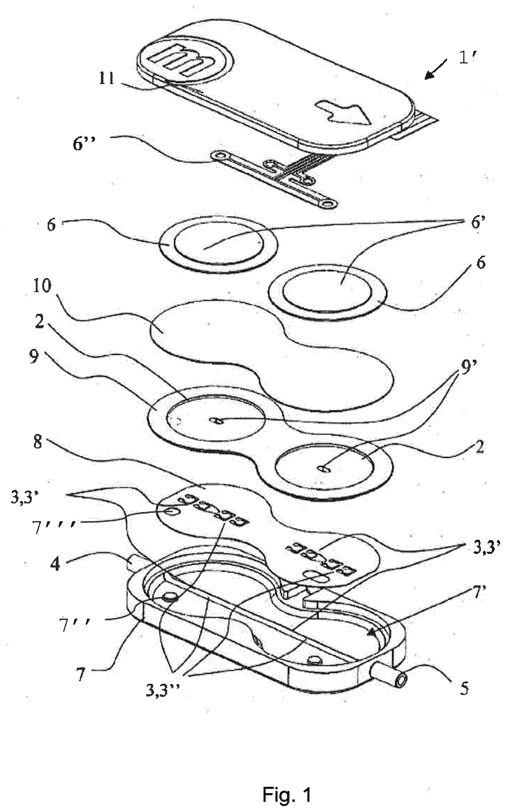

**Fig. 1** shows an exploded view of the Bartels mp6 micropump as described in the US patent application US 2011/0005606. The micropump 1' consists of a layered assembly, which comprises two serially arranged pump chambers 2. In Fig. 1, the assembly 1' consists in detail of the following components: a base element 7, a valve foil 8, an intermediate layer 9, a protection layer 10, two actuators 6 and a lid element 11.

The base element 7 particularly preferred is made of plastics. The base element comprises a recess 7' into which all subsequent components are inserted or put onto. The base element also includes inlet 4 and exit 5 which are provided for the delivery of the fluid, and which, as depicted here, are for example designed as a hose-like connector. Other connector types which are adapted to the respective application are possible as well. The base element 7 also comprises parts of the fluid channels which are necessary for the valves 3 which are preferably produced by injection moulding, and therefore, in the same process as the base element itself. Furthermore, the base element 7 carries projecting parts 7" of the same as mounting aids in the form of geometric features, which are arranged in such a manner that they cooperate with the recesses of the mounting aid 7"'. Therefore, the assembly of subsequent components such as for example the valve foil 8 can only occur in one certain way, so that an incorrect mounting is excluded to a large extent.

The valve foil 8 carries the movable parts of the valves 3. The valve foil 8 is being inserted into the base element 7. In the embodiment shown, the valve foil 8 comprises the movable parts of the inlet valves 3' of each pump chamber, as well as the movable parts of the respective outlet valves 3''. Furthermore, the valve foil also comprises the recesses of the mounting aid 7'" which serve for a faultless insertion of the valve foil.

The intermediate layer 9 is preferably made of plastics. It is designed in such a manner that it can be inserted into the recess 7' of the base element 7. In the centre of each pump chamber 2 which is respectively formed by a recess in the intermediate layer, one respective opening 9' is located through which fluid can flow into the respective pump chamber or out of the same.

The protection layer 10 is applied onto the intermediate layer and thus fluidically borders the pump chamber to the above. Thus, the protection layer must be firmly connected with the intermediate layer 9, so that fluid can exit or flow over neither at its circumference nor in the region between the pump chambers. Preferably, penetration laser welding is being used therefore. Alternative production techniques are gluing, ultrasound welding, or mechanical clamping of the respective components.

Two actuators 6 are provided as disc shaped piezo-actuators in the embodiment shown. Each of the actuators is geometrically adapted to the pump chamber 2 which is arranged below, and it carries according electrodes 6' for the electrical contacting. Connected to these is an electric terminal 6" which can be led out of the housing of the assembly 1', and which provides a sufficient number of individual wires for the connection of each actuator 6.

The lid element 11 serves as a seal of the housing of the apparatus, which housing substantially consists of the base element 7. Preferably, the lid element is also fabricated from plastic and is designed such that it can be connected with the base element 7 by means of penetration laser welding.

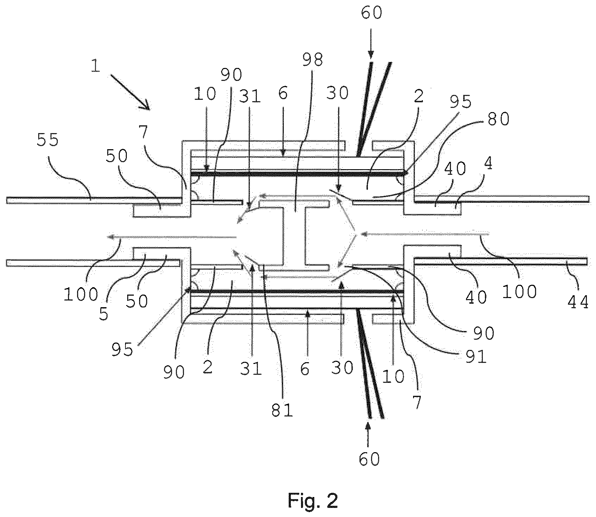

**Fig. 2** shows a micropump 1 with two actuators 6 arranged in parallel. The micropump is also a layered assembly, wherein the basic elements of one pump chamber may be similar as one individual pump chamber and actuator as described with respect to Fig. 1.

In Fig. 2, two base elements 7 each comprise a recess into which all components forming one pump chamber are inserted or put into. The two base elements 7 also preferably include half of an inlet 40 and half of an outlet 50, which upon assembly of the two base elements 7 form a common inlet 4 and a common outlet 5 as shown in Fig. 2. The inlet 4 and outlet 5 may be connected to tubings 44, 55, for example plastic hoses, for the delivery of fluid to the micropump and away from the micropump 1.

Two actuators 6 are provided as disc shaped piezo-actuators in the embodiment shown. One actuator each is inserted into a recess of a respective base element 7. The piezo-actuators may, for example, be a piezo ceramic mounted on a brass membrane, which piezo ceramic deforms the membrane when a voltage is applied to the piezo ceramic.

Each of the actuators 6 is geometrically adapted to the size of its recess, which basically defines the lateral extensions of the pump chambers 2 which are arranged below. The actuators 6 each are connected to electrodes and comprise wiring 60 for the electrical contacting of the actuators. The wires 60 lead out of the housing of the micropump 1.

A protection layer 10, for example a Kapton tape, is provided in the recess and forms one side of a pump chamber 2. The protection layer 10 transmits movement of the piezo actuator into the pump chamber 2. The protection layer 10 is held in place by a seal 95, for example a weld seal, clamping or an adhesive seal, also sealing the pump chamber 2.

An intermediate layer 90, preferably made of plastics, is each inserted into the respective recesses of the base elements 7. The intermediate layers 90 carry the valve foils and are held in place by another seal 95, such as for example a weld seal, also sealing the pump chamber 2. The space between intermediate layer 90 and protection layer 10 and the recess walls defines the size of the pump chamber 2.

Off center in the intermediate layer 90 in the direction of the inlet 4, an inlet opening 91 is located through which fluid can flow from the common inlet 4 into the respective pump chamber 2. Off center in the intermediate layer 90 into the direction of the outlet 5 an outlet opening 92 is located through which fluid can flow from the respective pump chamber 2 into the common outlet 5. In the center of the intermediate layer 90 a flow separation element 98 is arranged.

One flow separation element 98 is used for both pump chamber and is arranged between the two pump chambers separating the common inlet 4 from the common outlet 5 in view of a flow direction. A flow of fluid 100 from the common inlet 4 towards the common outlet 5 defines a fluid path, shown as the arrows in Fig. 2. The fluid path is shown comprising no angles of 90 degree or smaller, i.e. the flow path comprises only obtuse angles, because the flow separation element 98 has a shape and structure to support a smooth fluid flow from the common inlet 4 through the inlet valves 30 and from the outlet valves 31 into the common outlet 5.

A first valve foil 80 comprises the movable parts of the inlet valve or valves 30 of each pump chamber. A second valve foil 81 comprises the movable parts of the respective outlet valves 31. The first valve foil 80 may be attached to the intermediate layer 90. The second valve foil 81 may be attached to the flow separation element 98.

By parallel and synchronous actuation of the piezo actuators 6, a deformation thereof draws back the protective foils 10. Due to the generated under pressure the inlet valves 30 open and make the fluid flow 100 from the common inlet 4 to pass into the respective pump chambers 2. An actuation of the piezo actuators into the opposite direction compress the pump chambers 2 due to the flexibility of the protective layers 10 and push the fluid from the pump chambers 2 through the pushed open outlet valves 31 out of the pump chambers 2 and into the common outlet 5. Due to the arrangement of the valves, inlet valves 30 are automatically closed when outlet valves 31 are opened and vice versa.

Preferably, the pump chambers 2 have a same chamber volume and same chamber geometry. The micropump 1 of Fig. 1 is symmetric with respect to a virtual middle plane arranged between and parallel to the two actuators 6, and extending through the common inlet and common outlet. Such a construction allows the manufacture of the micropump with few parts only, preferably by two identical individual micropump halves comprising one base element and pump chamber.

## Claims

1. Aerosol-generating device comprising:
- a cartridge for holding an aerosol-forming substrate,
- an atomizer for atomizing aerosol-forming substrate;
- a micropump (1) for delivering a fluid, wherein the micropump (1) is arranged between the cartridge and the atomizer and in fluid connection with the cartridge and the atomizer for supplying aerosol-forming substrate from the cartridge to the atomizer, the micropump (1) comprising:
two pump chambers (2) having two separate chamber volumes;
two actuators (6), each actuator (6) assigned to one of the two pump chambers (2) for changing a respective chamber volume;
each pump chamber (2) provided with at least one inlet valve (30) and at least one outlet valve (31) for establishing a pumping direction along a fluid path, said fluid path comprising no angles of 90 degree or smaller;
a common inlet (4) and a common outlet (5),
wherein the two pump chambers (2) are arranged in parallel and are in fluid connection with the common inlet (4) and the common outlet (5),
and wherein the actuators (6) are configured to operate in parallel such that a volume change in each of the two pump chambers (2) occurs simultaneously for both pump chambers (2).

2. Device according to claim 1, wherein the two pump chambers (2) are in direct fluid connection with the common inlet (4) and the common outlet (5).

3. Device according to any one of the preceding claims, wherein the two pump chambers (2) as well as the two actuators (6) are arranged opposite each other.

4. Device according to any one of the preceding claims, wherein the two pump chambers (2) and the two actuators (6) are configured such that a same volume change in each of the two pump chambers (2) occurs upon operation of the two actuators (6).

5. Device according to any one of the preceding claims, wherein the chamber volumes of the two pump chambers (2) are identical.

6. Device according to any one of the preceding claims, wherein a flow rate is between 1 µL/s and 7 µL/s.

7. Device according to any one of the preceding claims, wherein the two actuators (6) are piezo membrane actuators.

8. Device according to any one of the preceding claims, wherein the micropump (1) comprises two inlet valves (30) and two outlet valves (31) per pump chamber (2).

9. Device according to any one of the preceding claims, wherein the micropump (1) comprises a symmetric set-up in view of a plane arranged parallel to and between the two pump chambers (2).

10. Device according to any one of the preceding claims, wherein an inlet connection of the common inlet (4) and an outlet connection of the common outlet (5) is arranged at a same side of the micropump (1).

11. Device according to any one of the preceding claims, further comprising a flow sensor connected to a control electronics for controlling a fluid flow in the common outlet (5) of the micropump (1).

12. Device according to any one of the preceding claims, wherein the atomizer comprises any one or a combination of an acoustic atomization element, an ultrasonic vibrator or a vaporizer such as a heater.

13. Device according to any one of the preceding claims, comprising one or a combination of nicotine containing aerosol-forming substrate and tobacco flavour containing aerosol-forming substrate.

14. Device according to any one of the preceding claims, comprising a viscous liquid aerosol-forming substrate comprising a viscosity between 1 mPas and 200 mPas, preferably between 1 mPas and 150 mPas.

15. Use of the device according to any one of the preceding claims in an electronic smoking system.

## Patentansprüche

1. Aerosolerzeugungsvorrichtung, aufweisend:
- eine Patrone zum Aufnehmen eines aerosolbildenden Substrats,
- einen Zerstäuber zum Zerstäuben von aerosolbildendem Substrat;
- eine Mikropumpe (1) zum Abgeben eines Fluids, wobei die Mikropumpe (1) zwischen der Patrone und dem Zerstäuber und in Fluidverbindung mit der Patrone und dem Zerstäuber angeordnet ist, um aerosolbildendes Substrat aus der Patrone dem Zerstäuber zuzuführen, wobei die Mikropumpe (1) aufweist:
zwei Pumpenkammern (2) mit zwei getrennten Kammervolumina;
zwei Aktoren (6), wobei jeder Aktor (6) einer der zwei Pumpenkammern (2) zugeordnet ist, um ein jeweiliges Kammervolumen zu verändern;
wobei jede Pumpenkammer (2) mit mindestens einem Einlassventil (30) und mindestens einem Auslassventil (31) versehen ist, um eine Pumprichtung entlang eines Fluidwegs festzulegen, und der Fluidweg keine Winkel von 90 Grad oder kleiner aufweist;
einen gemeinsamen Einlass (4) und einen gemeinsamen Auslass (5),
wobei die zwei Pumpenkammern (2) parallel angeordnet sind und in Fluidverbindung mit dem gemeinsamen Einlass (4) und dem gemeinsamen Auslass (5) stehen,
und wobei die Aktoren (6) ausgelegt sind, parallel zu arbeiten, sodass eine Volumenänderung in jeder der zwei Pumpenkammern (2) gleichzeitig für beide Pumpenkammern (2) erfolgt.

2. Vorrichtung nach Anspruch 1, wobei die zwei Pumpenkammern (2) in direkter Fluidverbindung mit dem gemeinsamen Einlass (4) und dem gemeinsamen Auslass (5) stehen.

3. Vorrichtung nach einem der vorstehenden Ansprüche, wobei die zwei Pumpenkammern (2) sowie die zwei Aktoren (6) einander gegenüberliegend angeordnet sind.

4. Vorrichtung nach einem der vorstehenden Ansprüche, wobei die zwei Pumpenkammern (2) und die zwei Aktoren (6) derart ausgelegt sind, dass bei Betätigung der zwei Aktoren (6) eine gleiche Volumenänderung in jeder der zwei Pumpenkammern (2) erfolgt.

5. Vorrichtung nach einem der vorstehenden Ansprüche, wobei die Kammervolumina der zwei Pumpenkammern (2) identisch sind.

6. Vorrichtung nach einem der vorstehenden Ansprüche, wobei eine Strömungsgeschwindigkeit zwischen 1 µL/s und 7 µL/s liegt.

7. Vorrichtung nach einem der vorstehenden Ansprüche, wobei die zwei Aktoren (6) Piezomembranaktoren sind.

8. Vorrichtung nach einem der vorstehenden Ansprüche, wobei die Mikropumpe (1) zwei Einlassventile (30) und zwei Auslassventile (31) pro Pumpenkammer (2) aufweist.

9. Vorrichtung nach einem der vorstehenden Ansprüche, wobei die Mikropumpe (1) im Hinblick auf eine parallel zu und zwischen den beiden Pumpenkammern (2) angeordnete Ebene eine symmetrische Anordnung aufweist.

10. Vorrichtung nach einem der vorstehenden Ansprüche, wobei ein Einlassanschluss des gemeinsamen Einlasses (4) und ein Auslassanschluss des gemeinsamen Auslasses (5) auf einer gleichen Seite der Mikropumpe (1) angeordnet sind.

11. Vorrichtung nach einem der vorstehenden Ansprüche, weiter aufweisend einen Strömungssensor, der mit einer Steuerelektronik zum Steuern einer Fluidströmung in dem gemeinsamen Auslass (5) der Mikropumpe (1) verbunden ist.

12. Vorrichtung nach einem der vorstehenden Ansprüche, wobei der Zerstäuber eines oder eine Kombination aus einem akustischen Zerstäubungselement, einem Ultraschallschwinger oder einem Verdampfer, wie beispielsweise eine Heizvorrichtung, aufweist.

13. Vorrichtung nach einem der vorstehenden Ansprüche, die eines oder eine Kombination von nikotinhaltigem aerosolbildendem Substrat und tabakgeschmackhaltigem aerosolbildendem Substrat aufweist.

14. Vorrichtung nach einem der vorstehenden Ansprüche, aufweisend ein viskoses flüssiges aerosolbildendes Substrat, das eine Viskosität zwischen 1 MPa und 200 MPa und bevorzugt zwischen 1 MPa und 150 MPa aufweist.

15. Gebrauch der Vorrichtung nach einem der vorstehenden Ansprüche in einem elektronischen System zum Rauchen.

## Revendications

1. Dispositif de génération d'aérosol comprenant :
- une cartouche pour maintenir un substrat formant aérosol,
- un atomiseur pour l'atomisation du substrat formant aérosol ;
- une micropompe (1) pour délivrer un fluide, dans lequel la micropompe (1) est agencée entre la cartouche et l'atomiseur et en connexion fluidique avec la cartouche et l'atomiseur pour fournir un substrat formant aérosol de la cartouche à l'atomiseur, la micropompe (1) comprenant :
deux chambres de pompe (2) ayant deux volumes de chambre séparés ;
deux actionneurs (6), chaque actionneur (6) affecté à l'une des deux chambres de pompe (2) pour modifier un volume de chambre respectif ;
chaque chambre de pompe (2) pourvue d'au moins une soupape d'entrée (30) et d'au moins une soupape de sortie (31) pour établir une direction de pompage le long d'un trajet de fluide, ledit trajet de fluide ne comprenant aucun angle de 90 degrés ou plus petit ;
une entrée commune (4) et une sortie commune (5),
dans lequel les deux chambres de pompe (2) sont agencées en parallèle et sont en connexion fluidique avec l'entrée commune (4) et la sortie commune (5),
et dans lequel les actionneurs (6) sont configurés pour fonctionner en parallèle de telle sorte qu'un changement de volume dans chacune des deux chambres de pompe (2) se produit simultanément pour les deux chambres de pompe (2).

2. Dispositif selon la revendication 1, dans lequel les deux chambres de pompe (2) sont en connexion de fluidique directe avec l'entrée commune (4) et la sortie commune (5) .

3. Dispositif selon l'une quelconque des revendications précédentes, dans lequel les deux chambres de pompe (2) ainsi que les deux actionneurs (6) sont agencés l'un face à l'autre.

4. Dispositif selon l'une quelconque des revendications précédentes, dans lequel les deux chambres de pompe (2) et les deux actionneurs (6) sont configurés de telle sorte qu'un changement de volume identique dans chacune des deux chambres de pompe (2) se produit lors du fonctionnement des deux actionneurs (6).

5. Dispositif selon l'une quelconque des revendications précédentes, dans lequel les volumes de chambre des deux chambres de pompe (2) sont identiques.

6. Dispositif selon l'une quelconque des revendications précédentes, dans lequel un débit est compris entre 1 pL/s et 7 µL/s.

7. Dispositif selon l'une quelconque des revendications précédentes, dans lequel les deux actionneurs (6) sont des actionneurs à membrane piézoélectrique.

8. Dispositif selon l'une quelconque des revendications précédentes, dans lequel la micropompe (1) comprend deux vannes d'entrée (30) et deux vannes de sortie (31) par chambre de pompe (2).

9. Dispositif selon l'une quelconque des revendications précédentes, dans lequel la micropompe (1) comprend une configuration symétrique en vue d'un plan disposé parallèlement et entre les deux chambres de pompe (2).

10. Dispositif selon l'une quelconque des revendications précédentes, dans lequel une connexion d'entrée de l'entrée commune (4) et une connexion de sortie de la sortie commune (5) sont disposées d'un même côté de la micropompe (1).

11. Dispositif selon l'une quelconque des revendications précédentes, comprenant en outre un capteur de débit connecté à un circuit électronique de contrôle pour contrôler un flux de fluide dans la sortie commune (5) de la micropompe (1).

12. Dispositif selon l'une quelconque des revendications précédentes, dans lequel l'atomiseur comprend l'un quelconque ou une combinaison d'un élément d'atomisation acoustique, d'un vibrateur à ultrasons ou d'un vaporisateur tel qu'un radiateur.

13. Dispositif selon l'une quelconque des revendications précédentes, comprenant une ou une combinaison de substrat formant aérosol contenant de la nicotine et de substrat formant aérosol contenant de l'arôme de tabac.

14. Dispositif selon l'une quelconque des revendications précédentes, comprenant un substrat formant aérosol liquide visqueux comprenant une viscosité comprise entre 1 mPas et 200 mPas, de préférence entre 1 mPas et 150 mPas.

15. Utilisation du dispositif selon l'une quelconque des revendications précédentes dans un système de fumage électronique.
